Europäisches Patentamt

European Patent Office

Office européen des brevets

(19)

(11) Numéro de publication: **0 182 772**
A2

(12) # DEMANDE DE BREVET EUROPEEN

(21) Numéro de dépôt: 85870161.8

(51) Int. Cl.⁴: **A 61 K 9/22**

(22) Date de dépôt: 19.11.85

(30) Priorité: 22.11.84 BE 214044

(71) Demandeur: **BENFAR, Rue Emile Vandervelde 25B, B-7210 Cuesmes (BE)**

(43) Date de publication de la demande: **28.05.86 Bulletin 86/22**

(72) Inventeur: **Vanderlinden, Pierre, Rue Emile Vandervelde 25B, B-7210 Cuesmes (BE)**

(84) Etats contractants désignés: **AT CH DE FR GB IT LI LU NL**

(74) Mandataire: **Schmitz, Yvon et al, Bureau Gevers S.A. 7, rue de Livourne Bte 1, B-1050 Bruxelles (BE)**

(54) Compositions pharmaceutiques à libération prolongée, leur procédé de préparation et leur utilisation.

(57) Compositions pharmaceutiques du type à libération prolongée destinées à l'administration par voie orale comprenant une substance pharmacologiquement active et un excipient pharmacologiquement acceptable, dans lesquelles l'excipient est constitué d'un mélange d'au moins de diméthylpolysiloxane, d'acide silicique, de mannanes et/ou de galactanes, de xanthanes et d'algues micronisées.

EP 0 182 772 A2

ACTORUM AG

"Compositions pharmaceutiques à libération prolongée, leur procédé de préparation et leur utilisation".

La présente invention est relative à des
compositions pharmaceutiques à administrer par voie
orale et dont le ou les principes actifs se libèrent de façon lente et régulière, à leur procédé de
préparation et à leur utilisation.

L'intérêt qui se manifeste actuellement
en thérapeutique vis-à-vis de ces formes galéniques
dites "à libération prolongée" provient des avantages qu'elles présentent par rapport aux formes conventionnelles, lesquelles libèrent leur principe actif
de façon plus rapide ou plus brutale.

Parmi ces avantages, on citera, notamment :

- leur aptitude à produire dans l'organisme des
  concentrations en principe actif plus uniformes
  dans le temps en éliminant les effets "en dents
  de scie" caractérisés par des pics où la concentration en médicament excède  le niveau thérapeutique
  et engendre des effets secondaires et des vallées
  où cette même concentration s'abaisse en-dessous
  de la zone efficace;
- la possibilité qu'elles offrent d'assurer une imprégnation médicamenteuse suffisante à l'organisme
  pendant les périodes où les prises de médicament sont irrégulières voire absentes, par exemple pendant les périodes nocturnes et pour des médicaments à courte durée d'action;

- la diminution des variations interindividuelles des taux sanguins en médicament, obtenues par la prolongation et la régularisation de la résorption;

- la réduction du nombre de prises journalières, ce qui simplifie la posologie et entraîne une meilleure observance du patient vis-à-vis de cette posologie;

- la possibilité d'utiliser des doses élevées de médicament tout en minimisant le risque d'atteindre les concentrations toxiques, en particulier dans les cas de médicaments dont l'indice thérapeutique est peu élevé.

Actuellement, les types de compositions les plus utilisées dans le domaine de la libération prolongée du ou des principes actifs sont les comprimés et les microgranules.

La présente invention a pour but de prévoir une composition pharmaceutique à libération prolongée destinée à l'administration par voie orale comprenant une substance pharmacologiquement active et un excipient assurant une libération plus lente, plus régulière, plus progressive et plus durable de la substance thérapeutique active après administration, que les compositions pharmaceutiques du même type connues jusqu'à présent.

A cet effet, suivant l'invention, l'excipient pharmacologiquement acceptable est constitué d'un mélange d'au moins de diméthylpolysiloxane, d'acide silicique, de mannanes et/ou de galactanes, de xanthanes et d'algues micronisées.

Avantageusement, le mélange susdit comprend de 15 à 50 parties en poids de diméthylpolysiloxane, de 30 à 100 parties en poids d'acide silicique, de 30 à 100 parties en poids de mannanes ou de galactanes et/ou d'un mélange de mannanes et de galactanes, de 50 à 150 parties en poids de xanthanes et de 25 à 75 parties en poids d'algues micronisées.

Suivant une forme de réalisation particulièrement avantageuse de l'invention, le mélange précité comprend environ 25 parties en poids de diméthylpolysiloxane, 50 parties en poids d'acide silicique, 50 parties en poids de mannanes ou de galactanes et/ou d'un mélange de manannes et de galactanes, 75 parties en poids de xanthanes et 50 parties en poids d'algues micronisées.

Suivant une réalisation particulièrement avantageuse de l'invention, l'excipient comprend un mélange de mannanes et de galactanes.

L'invention se rapporte également à la préparation de ces compositions pharmaceutiques à libération prolongée ainsi qu'à leur méthode d'utilisation, qui consiste à administrer par la voie orale ladite composition à des doses thérapeutiquement efficaces.

Comme on vient de le mentionner précédemment, la composition pharmaceutique de la présente invention est constituée d'une substance pharmacologiquement active et d'un excipient pharmacologiquement acceptable formé d'un mélange comprenant de

4     0182772

15 à 50 parties en poids et de préférence 25 parties en poids de diméthylpolysiloxane, de 30 à 100 parties en poids et de préférence 50 parties en poids d'acide silicique, de 30 à 100 parties en poids et de préférence 50 parties en poids de mannanes ou de galactanes ou d'un mélange de mannanes et de galactanes, de 50 à 150 parties en poids et de préférence 75 parties en poids de xanthanes et de 25 à 75 parties en poids et de préférence 50 parties en poids d'algues micronisées.

On obtient des résultats particulièrement intéressants lorsque l'on utilise un mélange de mannanes et de galactanes.

En fait, l'excipient de la présente invention a la propriété de former dans l'estomac un hydrocolloïde dont la digestion, sans apport calorique, se prolonge dans le temps, en provoquant ainsi une absorption ralentie du ou des principes actifs. Ce ralentissement de la digestion du ou des principes actifs est en fait provoqué principalement par l'effet de synergie qu'assurent les mannanes, les galactanes ou le mélange de mannanes et de galactanes sur les xanthanes. Les galactanes, mannanes et xanthanes sont des polysaccharides bien connus d'origine animale ou végétale. Ainsi qu'on l'a déjà précisé précédemment, on obtient des résultats particulièrement intéressants lorsque l'excipient comprend un mélange de mannanes et de galactanes. L'acide silicique quant à lui sert en fait de support au diméthylpolysiloxane, qui est un liquide visqueux qui vient s'adsorber sur celui-ci, et qui est un ingrédient de base pour pommade ou un véhicule de médicament à application topique bien connu en pharmacie. Le diméthylpolysiloxane 500 convient particulièrement bien à cet effet.

L'avantage des préparations ou formes posologiques unitaires de l'invention, telles que comprimés, capsules,granules , du point de vue médical consiste donc dans la possibilité de réduire fortement les doses de produit actif au bénéfice de la santé du patient. On pourrait également utiliser les compositions de l'invention sous la forme de vaccins.

Le rapport pondéral de l'excipient à la substance pharmacologiquement acceptable des compositions pharmaceutiques de l'invention est de préférence de l'ordre de 1/5 à 5/1, ces compositions contenant, par exemple, de 5 à 500 mg de substance active par dose unitaire.

Comme principe actif utilisable, on peut citer toutes les substances solides, ou rendues telles, administrables par la voie orale et dont les caractéristiques pharmacologiques et/ou pharmacocinétiques sont telles qu'il y a avantage à les présenter sous une forme à libération prolongée, et telles que, par exemple, les dérivés xanthiques, les antiinflammatoires, les β-bloquants, les inhibiteurs calciques, les antiépileptiques , les benzodiazépines, les diurétiques , les analgésiques, certaines substances hormonales, les antidépresseurs ,cette énumération étant non limitative.

On obtient les compositions pharmaceutiques à libération prolongée de l'invention en mélan-

geant la substance pharmacologiquement active et l'excipient,en les tamisant, en les granulant éventuellement et en les comprimant, pour obtenir par exemple un comprimé de poids et de dureté voulus.

Il doit être entendu que la présente invention n'est en aucune façon limitée aux formes de réalisation ci-dessus et que bien des modifications peuvent y être apportées sans sortir du cadre du présent brevet.

REVENDICATIONS.

1. Composition pharmaceutique du type à libération prolongée destinée à l'administration par voie orale comprenant une substance pharmacologiquement active et un excipient pharmacologiquement acceptable, caractérisée en ce que l'excipient est constitué d'un mélange d'au moins de diméthylpolysiloxane, d'acide silicique, de mannanes et/ou de galactanes, de xanthanes et d'algues micronisées.

2. Composition suivant la revendication 1, caractérisée en ce que le mélange susdit comprend de 15 à 50 parties en poids de diméthylpolysiloxane, de 30 à 100 parties en poids d'acide silicique, de 30 à 100 parties en poids de mannanes ou de galactanes ou d'un mélange de mannanes et de galactanes, de 50 à 150 parties en poids de xanthanes et de 5 à 75 parties en poids d'algues micronisées.

3. Composition suivant la revendication 2, caractérisée en ce que le mélange précité comprend environ 25 parties en poids de diméthylpolysiloxane, 50 parties en poids d'acide silicique, 50 parties en poids de mannanes ou de galactanes ou d'un mélange de mannanes et de galactanes, 75 parties en poids de xanthanes et 50 parties en poids d'algues micronisées.

4. Composition suivant l'une quelconque des revendications 1 à 3, caractérisée en ce que le mélange précité comprend un mélange de mannanes et de galactanes.

5. Composition suivant l'une quelconque des revendications 1 à 4, caractérisée en ce que le diméthylpolysiloxane est du diméthylpolysiloxane 500.

6. Composition suivant l'une quelconque des revendications 1 à 5, caractérisée en ce qu'elle est sous une forme posologique unitaire, telle que comprimé, capsule, granule.

7. Composition suivant l'une quelconque des revendications 1 à 6, caractérisée en ce que le rapport pondéral de l'excipient à la substance pharmacologiquement acceptable est de l'ordre de 1/5 à 5/1.

8. Composition suivant l'une ou l'autre des revendications 6 et 7, caractérisée en ce qu'elle contient de 5 à 500 mg de substance active par dose unitaire.

9. Composition suivant l'une quelconque des revendications 1 à 5, caractérisée en ce qu'elle est sous la forme de vaccin.

10. Composition suivant l'une quelconque des revendications 1 à 9, caractérisée en ce que la substance pharmacologiquement active est choisie dans le groupe comprenant les dérivés xanthiques, les antiinflammatoires, les $\beta$-bloquants, les inhibiteurs calciques, les antiépileptiques, les benzodiazépines, les diurétiques, les analgésiques, les hormones et les antidépresseurs.

11. Procédé de préparation de la composition pharmaceutique du type à libération prolongée suivant l'une quelconque des revendications 1 à 8 et 10, caractérisé en ce que l'on mélange la substance pharmacologiquement active et l'excipient, les tamise, les granule éventuellement et les comprime.

12. Méthode d'utilisation de la composition suivant l'une quelconque des revendications 1 à 10, caractérisée en ce qu'on l'administre par voie orale à des doses thérapeutiquement efficaces.

REVENDICATIONS. (AUTRICHE)

1. Procédé de préparation d'une composition pharmaceutique du type à libération prolongée destinée à l'administration par voie orale comprenant une substance pharmacologiquement active et un excipient pharmacologiquement acceptable, caractérisé en ce que l'on mélange la substance pharmacologiquement active et l'excipient, cet excipient étant constitué d'un mélange d'au moins de diméthylpolysiloxane, d'acide silicique, de mannanes et/ou de galactanes, de xanthanes et d'algues micronisées.

2. Procédé suivant la revendication 1, caractérisé en ce que l'on tamise, granule éventuellement et comprime la substance pharmacologiquement active et l'excipient pharmacologiquement acceptable.

3. Procédé suivant l'une ou l'autre des revendications 1 et 2, caractérisé en ce que le mélange susdit comprend de 15 à 50 parties en poids de diméthylpolysiloxane, de 30 à 100 parties en poids d'acide silicique, de 30 à 100 parties en poids de mannanes ou de galactanes ou d'un mélange de mannanes et de galactanes, de 50 à 150 parties en poids de xanthanes et de 5 à 75 parties en poids d'algues micronisées.

4. Procédé suivant la revendication 3, caractérisé en ce que le mélange précité comprend environ 25 parties en poids de diméthylpolysiloxane, 50 parties en poids d'acide silicique, 50 parties en poids de mannanes ou de galactanes ou d'un mélange de mannanes et de galactanes, 75 parties en poids de xanthanes et 50 parties en poids d'algues micronisées.

8

0182772

5. Procédé suivant l'une quelconque des revendications 1 à 4, caractérisé en ce que le mélange précité comprend un mélange de mannanes et de galactanes.

6. Procédé suivant l'une quelconque des revendications 1 à 5, caractérisé en ce que le diméthylpolysiloxane est du diméthylpolysiloxane 500.

7. Procédé suivant l'une quelconque des revendications 1 à 6, caractérisé en ce que la composition est sous une forme posologique unitaire, telle que comprimé, capsule, granule.

8. Procédé suivant l'une quelconque des revendications 1 à 7, caractérisé en ce que le rapport pondéral de l'excipient à la substance pharmacologiquement acceptable est de l'ordre de 1/5 à 5/1.

9. Procédé suivant l'une ou l'autre des revendications 7 et 8, caractérisé en ce que la composition contient de 5 à 500 mg de substance active par dose unitaire.

10. Procédé suivant l'une quelconque des revendications 1 et 3 à 6, caractérisé en ce que la composition est sous la forme de vaccin.

11. Procédé suivant l'une quelconque des revendications 1 à 10, caractérisé en ce que la substance pharmacologiquement active est choisie dans le groupe comprenant les dérivés xanthiques, les antiinflammatoires, les $\beta$-bloquants, les inhibiteurs calciques, les antiépileptiques, les benzodiazépines, les diurétiques, les analgésiques, les hormones et les antidépresseurs.

12. Méthode d'utilisation de la composition préparée par le procédé suivant l'une quelconque des revendications 1 à 11, caractérisée en ce qu'on l'administre par voie orale à des doses thérapeutiquement efficaces.